# EUROPEAN PATENT SPECIFICATION

(11) **EP 1 948 779 B1**
(45) Date of publication and mention of the grant of the patent: **08.07.2015**
(21) Application number: 06826977.8
(22) Date of filing: 26.10.2006
(51) Int. Cl.: B01F 3/04, B01F 7/00, B01F 7/16, B01F 13/08, B01F 15/00, C12M 1/00, C12M 1/06, C12M 1/34

(54) **APPARATUS FOR BIOPROCESSING**
VORRICHTUNG FÜR BIOLOGISCHE VERFAHREN
SYSTEME DE TRAITEMENT BIOLOGIQUE

(30) Priority: 26.10.2005 US 730489 P; 30.08.2006 US 841012 P
(43) Date of publication of application: 30.07.2008
(73) Proprietor: Pall Technology UK Limited, Portsmouth, PO6 4BQ (GB)
(72) Inventor: TERENTIEV, Alexandre, N., Lexington, KY 40515 (US); TERENTYEV, Sergey, Lexington, KY 40502 (US)
(74) Representative: Lambert, Ian Robert
(86) International application number: PCT/US2006/042161
(87) International publication number: WO 2007/050971

(56) References cited:
- EP-A1- 0 172 478
- EP-A2- 1 473 358
- EP-A2- 1 479 758
- EP-A2- 1 961 806
- DE-A1- 19 705 118
- GB-A- 2 202 549
- JP-A- 61 212 276
- US-A- 1 452 966
- US-A- 2 404 679
- US-A- 3 384 354
- US-A- 3 854 704
- US-A- 3 900 186
- US-A- 4 204 774
- US-A- 4 649 118
- US-A- 4 870 018
- US-A1- 2002 105 856
- US-B2- 6 494 613
- US-B2- 6 670 171

## Description

### Technical Field

The present invention relates generally to vessels for holding fluids and, more particularly, to a bioreactor including a mixer and a sparger.

### Background of the Invention

Typically, a bioreactor comprises a sterile vessel designed to provide optimum growth conditions for a cell culture. To create such optimum conditions, the cell culture in the bioreactor often needs to be mixed during cell growth. Also, gases like oxygen need to be delivered to cell culture to maintain proper conditions for cell metabolism, pH and dissolved oxygen probes are normally used to control and maintain parameters at the optimal levels.

Usually, mixing impellers uniformly disperse gas, such as in the form of bubbles, throughout the volume of bioreactor. The gas bubbles may be formed by introducing pressurized gas to the fluid through a sparger or sparging element, which usually has small holes or pores that break the gas incoming gas into fine bubbles. Since small bubbles have a large surface to volume ratio, diffusion of the gas into fluid is greatly accelerated.

Traditional bioreactors comprise vessels made of stainless steel or glass. However, the current trend in biopharmaceutical manufacturing is to switch from such vessels to more readily disposable ones and, in particular, flexible plastic bags. When sterilized, disposable bags eliminate time consuming cleaning and validation, resulting in reduced cross contamination risk. Storage and transportation costs are also reduced.

A mixing bag including a sleeve and rotational rigid rod introduced inside the sleeve is described in commonly assigned U.S. Patent No. 6,494,613. The rigid rod introduced into the tube or sleeve is rotatedby an external motor to create mixing action inside the bag.

However, mixing alone is insufficient to operate the bag in bioreactor regime.

European Patent Publication No. 1473358A2 describes a method and system for introducing gas to a material within a rigid container and mixing the fluid with the container by means of a rotating control shaft carrying a number of paddles. However, a need is identified for an improved bioreactor for use in bioprocessing applications. The bioreactor would be easy to construct in an expensive fashion, and would be readily disposable and otherwise simple to use. Despite its simplicity, the apparatus provided would result in an unprecedented level of mixing ability, including possibly under sterile conditions, while at the same time facilitating cell growth by ensuring the full distribution of bubbles from any sparger provided throughout the fluid to improve the concentration of dissolved gas.

### Summary of the Invention

One aspect of the invention is an apparatus intended for use in bioprocessing with a fluid. The apparatus comprises a vessel having an interior compartment capable of holding the fluid; a mixer rotatable about an axis of rotation to agitate the fluid, a sparger for supplying a gas to the fluid; and a motive device for moving the sparger about the axis of rotation, whereby the mixer creates a mixing action in the fluid and the movement of the sparger about the axis of rotation helps distribute the sparger throughout the fluid to improve the concentration of dissolved gas; characterised in that the vessel is a bag including a flexible sleeve projecting within the interior compartment, and in that the mixer is positioned within the sleeve. The mixer comprises a mixing rod that induces rotation in the sleeve to agitate the fluid without exposing the mixing rod to the fluid. In any of these embodiments, the sparger may comprise an elongated flexible tube for delivering the gas to the fluid in the vessel by way of a porous material, and the mixer may include rigid blades.

A mixer is provided for insertion in the sleeve that induces rotation in the sleeve to agitate the fluid without exposing the mixer to the fluid without causing the sleeve to bodily rotate about a longitudinal axis. A sparger is also provided for forming bubbles from a gas supplied to the fluid in the vessel. As a result of this arrangement, the mixing action in the fluid created by the mixer helps distribute the bubbles from the sparger throughout the fluid to improve the concentration of dissolved gas.

Preferably, a weld connects the sparger to the vessel to form the static seal, and the sparger lies adjacent a floor of the vessel. Most preferably, the sparger couples to the flexible sleeve, and comprises an elongated flexible tube for delivering the gas to the fluid in the vessel by way of a porous material. The vessel also preferably includes at least one port in communication with the elongated flexible tube, which may be external to the interior compartment.

In one embodiment, the bioreactor further comprises a second mixer for agitating the fluid. Preferably, the second mixer comprises an impeller adjacent a floor of the vessel. To enhance the mixing action provided, the sleeve may carry rigid blades. Furthermore, the vessel may comprise a flexible bag to promote disposability.

### Brief Description of the Drawings

Figure 1 is a partially cutaway perspective view of the bioreactor vessel according to one embodiment of the invention;
Figure 2 is a partially cutaway perspective view of a bioreactor vessel according to another embodiment of the invention;
Figure 2a is a partially cutaway, enlarged, cross-sectional view of the vessel Figure 2; and
Figure 4 is a partially cutaway perspective view of a bioreactor vessel according to yet another embodiment of the invention.

### Detailed Description of the Invention

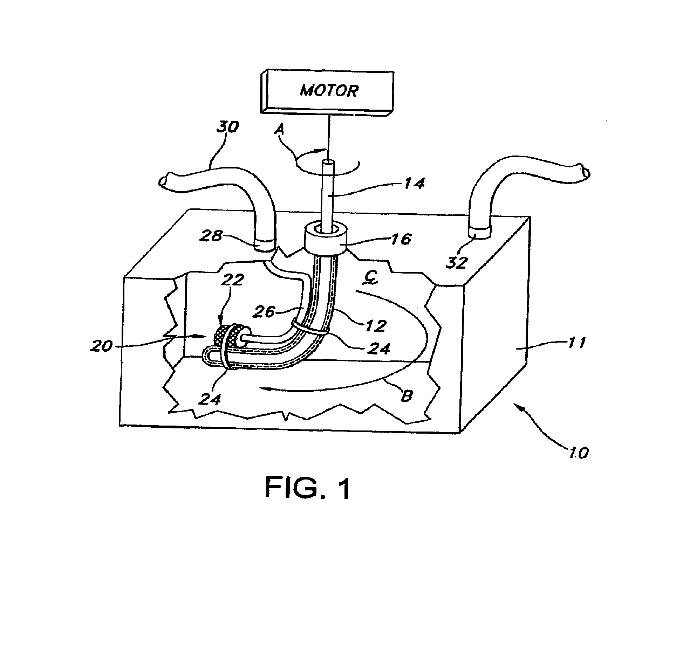

Reference is now made to Figure 1, which discloses one embodiment of the vessel 10 of the present invention for use as a bioreactor. The vessel 10 in this embodiment comprises a collapsible bag 11 including an inner compartment C for receiving and holding a fluid (which term incorporates liquids, gases, solid suspensions, and the like) for being agitated or mixed. As described in the above reference '613 patent, in the case of a collapsible bag 11, support may be provided by an outer rigid container (not shown so as to provide support for the fluid as well. However, it is within the broadest aspects of the invention for the vessel 10 to itself comprise a rigid container made of any suitable material, including rigid plastic, glass, metal, or the like.

In the embodiment shown in Figure 1, a flexible sleeve 12 projects in the interior compartment of the vessel 10 or bag 11 for agitating any fluid present. The sleeve 12 includes a closed end in the compartment C so as to form a cavity within the vessel 10 or bag 11 and an open end for receiving a mixer, such as a rigid mixing rod 14 having at least some degree of curvature. Adjacent the open end, the sleeve 12 is coupled to the vessel 10 or bag 11 and projects therein by way of a seal 16. This seal 16 is preferably hermetic, and may be statically formed by a circumferential weld formed between the open end of the sleeve 12 and an opening or hole in the vessel 10 or bag 11 so as to give it an annular shape. As a result of this type of attachment, the sleeve 12 is incapable of bodily rotating about its own longitudinal axis, but can still move about the interior compartment C in order to agitate the fluid and provide the desired mixing action (note action arrow B).

The distal end of the mixing rod 14 maybe inserted into the sleeve 12 so as to engage the distal end thereof, and may be supported by optional spacers as disclosed in the '613 patent. The opposite end of the rod 14 may in turn couple with or connect to a motive device, such as a rotational motor. Upon being actuated, this motor bodily rotates the rod 14 about its own longitudinal axis (note action arrow A). As a result of this movement, the sleeve 12 rotates about the interior compartment C to agitate the fluid, but without rotating about its own longitudinal axis. Moreover, by virtue of the sleeve 12 being closed within the interior compartment C, the mixing rod 14 never contacts the fluid directly, and can thus be withdrawn and reused in a different mixing application with this or a similar type of vessel 10 without being cleaned.

In accordance with one aspect of the invention, the bioreactor vessel 10 includes a movable sparger 20. In the embodiment of Figure 1, the sparger 20 comprises a porous material 22 connected to the distal end of the sleeve 12. The connection may be by way of a band 24, tie, fastener or like coupling means, or instead may be by way of welding or a like type of connection (e.g., an adhesive).

An internal conduit, which may be a rigid pipe but is shown as comprising a flexible tube 26, in turn connects the porous material 22 to a port 28 formed in the vessel 10 or bag 11. This tube 26 may also be coupled to the sleeve 12, such as by using another band 24, tie, or like coupler. The port 28, which may be hermetically sealed, couples with an external supply line, such as conduit 30, for supplying gas from a remote source (not shown) to the tube 26 and hence the porous material 22. However, it is within the broadest aspects of the invention to use any opening formed in the vessel 10 or bag 11 as the port 28 for receiving the flexible tube 26, which may in such case simply be coextensive with the supply line 30.

The porous material 22 is preferably such that it allows for the "one way" passage of fluid only; in other words, gas can pass through the material into an adjacent fluid, but the fluid and gas cannot pass through the porous material into the tube 26. Consequently, in the case where the vessel 10 or bag 11 is hermetically sealed, a vent or exhaust port 32 may also be provided. Preferably, a filter (not shown) or like means is associated with the port 32 for preventing the introduction of undesired contaminants, such as microorganisms or the like.

In operation, the rod 14 of the mixer is rotated to induce movement in the sleeve 12, which again does not rotate about its own longitudinal axis. This movement causes the porous material 22 of the sparger 20 to move simultaneously with the sleeve 12 about the interior compartment C and preferably adjacent the floor of the vessel 10. This movement helps to distribute the bubbles from the sparger 20 throughout the interior compartment C and thereby improves the concentration of dissolved gas in the fluid (which may be controlled by simply adjusting the supply of gas to the supply tube 30).

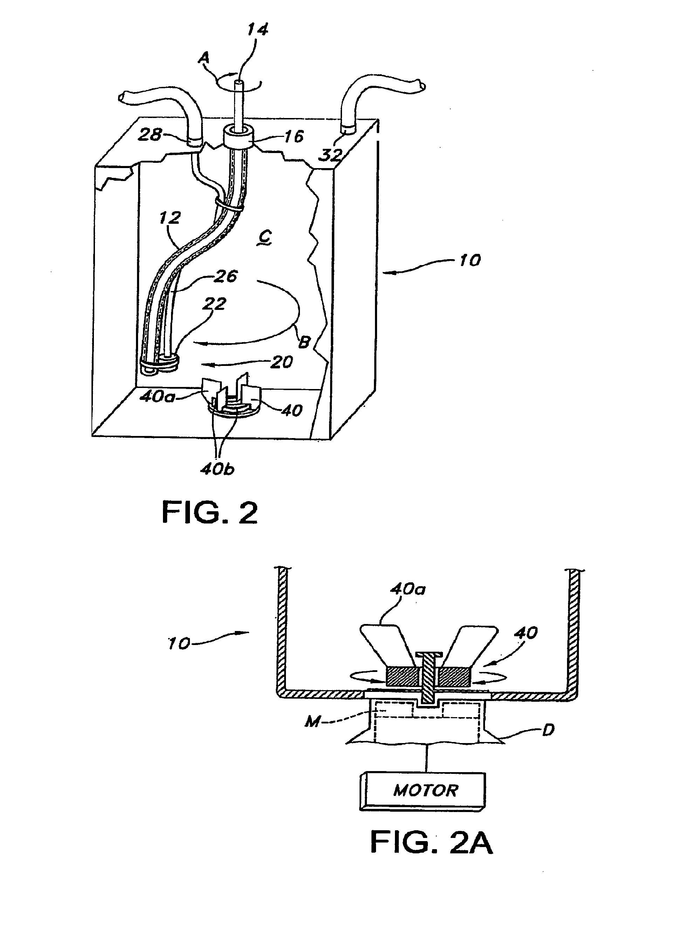

Figure 2 illustrates an alternative embodiment, which in many respects is similar to the one described in Figure 1. However, in addition to the agitation of the fluid created by the "wand" mixer, additional agitating capacity is provided by a second mixer positioned in the vessel 10. In the embodiment of Figure 2, this second mixer preferably comprises a rotatable stirrer and, most preferably, a bodily rotatable impeller 40, such as the one shown having one or more rigid blades 40a. Preferably, this impeller 40 lies adjacent a floor of the vessel 10, which again may be rigid or a flexible bag. The most preferred positioning is generally opposite the "wand" mixer in the illustrated embodiment, which thereby ensures that the agitation is provided in both the upper and lower portions of the internal compartment C.

While it is within the broadest aspects the invention for the second mixer to comprise a second wand mixer (not shown), the preferred use of a bodily rotatable impeller 40 may further comprise one or more magnets 40b, either alone or in combination with a matrix material. These magnets 40b may connect through the vessel 10 with an external motive device (note device D and drive magnet M) by way of a magnetic coupling, which coupling may then be used to induce rotation for agitating the fluid. The vessel 10 may further include a post P for receiving the impeller 40, and preferably a bearing providing support for it at least in a resting position. This bearing may comprise, for example, the peripheral seating surface shown as part of the rigid disc-shaped structure supporting the post P, or a separate roller bearing element supporting impeller 40. The impeller 40 may also be free of direct connection to the post P and levitated and/or rotated by a thermally shielded superconducting element in place of or in addition to the drive magnet M. In any case, this embodiment can be characterized as a collapsible, hermetically sealed vessel 10 having an interior compartment capable of receiving and holding the fluid, with first and second independently rotatable fluid-agitating elements for thoroughly mixing the fluid and/or enhancing the distribution of the bubbles from the sparger 20 throughout the fluid to improve the concentration of dissolved gas.

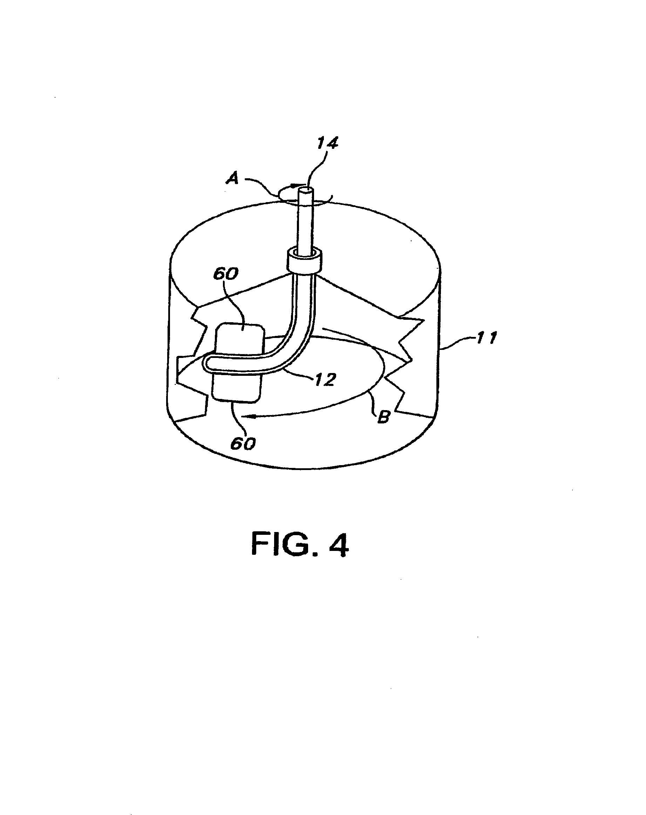

Figure 4 illustrates another embodiment of a vessel 10 including a rotational wand mixer similar in basic construction to the one shown in Figure 1. However, in this embodiment, at least one and preferably a pair of opposed rigid blades 60 attach directly to the flexible sleeve 12, such as by using a weld, fastener, or like means of connection.

Consequently, these blades 60 move about the fluid and help to enhance agitation as the sleeve 12 rotates about the interior compartment of the vessel 10 (such as the bag 11, which is shown as being cylindrical for purposes of illustration only), but without rotating about its own longitudinal axis (and thereby avoiding the need for a dynamic seal). Preferably, the blades 60 extend radially and, most preferably, in an opposed fashion, and may be spaced along the longitudinal axis of the sleeve 12. Sparging function may be provided by an optional sparger (not shown in Figure 4), which may be coupled to the sleeve 12 (as in Figures 1 and 2) or integral with the vessel 10.

Obvious modifications or variations are possible in light of the above teachings. For example, it may also be desirable to provide disposable means in the vessel 10 to facilitate sensing characteristics of the fluid, such as the pH, oxygen content, temperature, etc. As briefly noted above, the porous material 22 could also be integrally formed with the sleeve 12, as could the rigid blades 60.

## Claims

1. An apparatus for bioprocessing with a fluid, comprising:
a vessel (10) having an interior compartment capable of holding the fluid;
a mixer (14) rotatable about an axis of rotation to agitate the fluid:
a sparger (20) for supplying a gas to the fluid; and
a motive device for moving the sparger about the axis of rotation,
whereby the mixer (14) creates a mixing action in the fluid and the movement of the sparger (20) about the axis of rotation helps distribute the sparger throughout the fluid to improve the concentration of dissolved gas; **characterised in that** the vessel is a bag (11) including a flexible sleeve (12) projecting within the interior compartment, and **in that** the mixer (14) is positioned within the sleeve.

2. The apparatus of claim 1 wherein the mixer comprises a mixing rod (14) for insertion in the flexible sleeve (12) that induces rotation in the sleeve (12) to agitate the fluid without exposing the mixing rod (14) to the fluid.

3. The apparatus of claim 1. wherein the sleeve (12) carries rigid blades (40a).

4. The apparatus of claim 1, further including a second mixer for agitating the fluid.

5. The apparatus of claim 1, wherein the sparger (20) includes a tube (26) in communication at one end with a port in the vessel (10) and at the other end with a porous material for forming the bubbles from the gas supplied through the tube (26).

6. The apparatus of claim 1, wherein the motive device comprises a motor for mechanically coupling to the mixing rod (14).

7. The apparatus of claim 1, wherein the sparger (20) comprises a porous material connected to [[a]] the distal end of the sleeve.

8. The apparatus of claim 1, wherein the porous material is connected to the sleeve (12) by a band (24).

9. The apparatus of claim 5, wherein the tube (26) is coupled to the sleeve (12).

10. The apparatus of claim 12, further including an exhaust port (32) in the bag (11).

11. The apparatus of claim 10, wherein a filter is associated with the exhaust port (32) for preventing the introduction of undesired contaminants.

12. The apparatus of claim 1, wherein the sparger (20) comprises an elongated flexible tube for delivering the gas to the fluid in the vessel by way of a porous material.

13. The apparatus of claim 1, further including a hermetic seal statically formed by a circumferential weld formed between an open end of the sleeve (12) and an opening or hole in the bag (11) so as to give the seal an annular shape.

## Patentansprüche

1. Vorrichtung für biologische Verfahren mit einem Fluid, umfassend ein Gefäß (10), das eine innere Kammer aufweist, die das ,Fluid aufnehmen kann, ferner umfassend einen Mischer (14), der um eine Drehachse drehbar ist, um das Fluid umzuwälzen, des weiteren umfassend ein Einblasrohr (20) zur Zuführung eines Gases zu dem Fluid, und eine Antriebsvorrichtung, die dazu dient, das Einblasrohr um die Drehachse zu bewegen, wodurch der Mischer (14) in dem Fluid eine Mischwirkung entfaltet und die Bewegung des Einblasrohres (20) um die Drehachse hilft, das Einblasrohr in dem ganzen Fluid zur Geltung zu bringen, um dadurch die Konzentration des gelösten Gases zu verbessern, **dadurch gekennzeichnet, daß** das Gefäß ein Behälter (11) ist, der eine flexible Hülse (12) aufweist, die sich in die innere Kammer hinein erstreckt, und daß der Mischer (14) in der Hülse angeordnet ist.

2. Vorrichtung nach Anspruch 1, **dadurch gekennzeichnet, daß** der Mischer einen Mischstab (14) aufweist, der sich in die flexible Hülse (12) hinein erstreckt, um in der Hülse (12) eine Drehbewegung zu bewirken und dadurch das Fluid umzuwälzen, ohne daß der Mischstab (14) dem Fluid ausgesetzt wird.

3. Vorrichtung nach Anspruch 1, **dadurch gekennzeichnet, daß** die Hülse (12) steife Schaufeln (40a) trägt.

4. Vorrichtung nach Anspruch 1, **gekennzeichnet durch** einen zweiten Mischer zur Umwälzung des Fluids.

5. Vorrichtung nach Anspruch 1, **dadurch gekennzeichnet, daß** das Einblasrohr (20) ein Rohr (26) aufweist, das an dem einen Ende mit einer Öffnung in dem Gefäß (10) in Verbindung steht und an dem anderen Ende mit einem porösen Material in Verbindung steht, um von dem Gas, das durch das Rohr 26 zugeführt wird, Blasen zu erzeugen.

6. Vorrichtung nach Anspruch 1, **dadurch gekennzeichnet, daß** die Antriebsvorrichtung einen Motor aufweist, der mechanisch mit dem Mischstab gekoppelt ist.

7. Vorrichtung nach Anspruch 1, **dadurch gekennzeichnet, daß** das Einblasrohr (20) ein poröses Material aufweist, das mit dem entfernten Ende der Hülse verbunden ist.

8. Vorrichtung nach Anspruch 1, **dadurch gekennzeichnet, daß** das poröse Material mit der Hülse (12) durch ein Band (24) verbunden ist.

9. Vorrichtung nach Anspruch 5, **dadurch gekennzeichnet, daß** das Rohr (26) mit der Hülse (12) gekoppelt ist.

10. Vorrichtung nach Anspruch 1, **gekennzeichnet durch** eine Auslaßöffnung (32) in dem Behälter (11).

11. Vorrichtung nach Anspruch 10, **gekennzeichnet durch** einen Filter, der der Auslaßöffnung (32) zugeordnet ist und dazu dient, das Eindringen von unerwünschten Verunreinigungen zu verhindern.

12. Vorrichtung nach Anspruch 1, **dadurch gekennzeichnet, daß** das Einblasrohr (20) ein langgestrecktes, flexibles Rohr bildet, das dazu dient, das Gas mit Hilfe des porösen Materials in das in dem Gefäß befindliche Fluid abzugeben.

13. Vorrichtung nach Anspruch 1, ferner **gekennzeichnet durch** eine hermetische Dichtung, die statisch **durch** eine umfängliche Schweißnaht zwischen einem offenen Ende der Hülse (12) und einer Öffnung oder einem Loch in dem Behälter (11) so ausgebildet ist, daß die Dichtung eine ringförmige Gestalt erhält.

## Revendications

1. Appareil de traitement biologique avec un fluide, comprenant :
un récipient (10) ayant un compartiment intérieur capable de contenir le fluide ;
un mélangeur (14) apte à tourner autour d'un axe de rotation pour agiter le fluide ;
un aérateur (20) pour fournir un gaz au fluide ; et
un dispositif moteur pour déplacer l'aérateur autour de l'axe de rotation, ce par quoi le mélangeur (14) crée une action de mélange dans le fluide et le mouvement de l'aérateur (20) autour de l'axe de rotation aide à répartir l'agitateur à travers le fluide pour améliorer la concentration de gaz dissous ; **caractérisé par le fait que** le récipient est une poche (11) comprenant une gaine souple (12) se projetant à l'intérieur du compartiment intérieur, et **par le fait que** le mélangeur (14) est positionné à l'intérieur de la gaine.

2. Appareil selon la revendication 1, dans lequel le mélangeur comprend une tige de mélange (14) pour introduction dans la gaine souple (12), qui induit une rotation dans la gaine (12) pour agiter le fluide sans exposer la tige de mélange (14) au fluide.

3. Appareil selon la revendication 1, dans lequel la gaine (12) porte des lames rigides (40a).

4. Appareil selon la revendication 1, comprenant en outre un second mélangeur pour agiter le fluide.

5. Appareil selon la revendication 1, dans lequel l'aérateur (20) comprend un tube (26) en communication, à une extrémité, avec un orifice du récipient (10) et, à l'autre extrémité, avec une matière poreuse pour former les bulles à partir du gaz fourni à travers le tube (26).

6. Appareil selon la revendication 1, dans lequel le dispositif moteur comprend un moteur pour un accouplement mécanique à la tige de mélange (14).

7. Appareil selon la revendication 1, dans lequel l'aérateur (20) comprend une matière poreuse reliée à l'extrémité distale de la gaine.

8. Appareil selon la revendication 1, dans lequel la matière poreuse est reliée à la gaine (12) par une bande (24).

9. Appareil selon la revendication 5, dans lequel le tube (26) est accouplé à la gaine (12).

10. Appareil selon la revendication 12, comprenant en outre un orifice d'échappement (32) dans la poche (11).

11. Appareil selon la revendication 10, dans lequel un filtre est associé à l'orifice d'échappement (32) pour empêcher l'introduction de contaminants indésirables.

12. Appareil selon la revendication 1, dans lequel l'aérateur (20) comprend un tube souple allongé pour distribuer le gaz au fluide dans le récipient au moyen d'une matière poreuse.

13. Appareil selon la revendication 1, comprenant en outre un scellement hermétique formé statiquement par une soudure circonférentielle formée entre une extrémité ouverte de la gaine (12) et une ouverture ou un trou dans la poche (11) de façon à donner au scellement une forme annulaire.
